# EUROPÄISCHE PATENTANMELDUNG

(11) **EP 3 957 231 A1**
(43) Veröffentlichungstag der Anmeldung: **23.02.2022**
(21) Anmeldenummer: 20191999.0
(22) Anmeldetag: 20.08.2020
(51) Int. Cl.: A61B 5/00, H05K 1/02

(54) **FLEXIBLE LEITERPLATTE UND KATHETER MIT EINER SOLCHEN LEITERPLATTE**

(71) Anmelder: Freudenberg SE, 69469 Weinheim (DE)
(72) Erfinder: ALTUNCU, Sueleyman, 68305 Mannheim (DE); Rupp, David, Dr., 68623 Lampertheim (DE); Schmied, Benno, 67071 Ludwigshafen (DE)
(74) Vertreter: Fritz, Martin Richard

(57) **Zusammenfassung**

Die Erfindung betrifft eine flexible Leiterplatte (1) aus einem Streifen (2) für einen Katheter, wobei der Streifen (2) mehrere sich winkelig aneinander anschließende Abschnitte aufweist, derart, dass Umlenkungen (4.1, 4.2, 4.3) des Streifens (2) erfolgen, mit Faltkanten (3.1, 3.2) in dem Streifen (2) im Bereich der Umlenkungen (4.1, 4.2, 4.3), wobei durch Falten entlang der Faltkanten (3.1, 3.2) ein linearer gerader Streifen gebildet ist. Aufgabe der vorliegenden Erfindung ist es eine flexible Leiterplatte (1) zu schaffen, welche eine große Länge und gleichzeitig besonders kompakte Abmaße in ihrer Breite und Dicke aufweist. Erfindungsgemäß liegen im Bereich von drei Umlenkungen (4.1, 4.2, 4.3) zwei Faltkanten (3.1, 3.2) vor und maximal zwei Lagen des Streifens (2) aufeinander auf.

## Beschreibung

Die Erfindung betrifft eine flexible Leiterplatte aus einem Streifen für einen Katheter, wobei der Streifen mehrere sich winkelig aneinander anschließende Abschnitte aufweist, derart, dass Umlenkungen des Streifens erfolgen, mit Faltkanten in dem Streifen im Bereich der Umlenkungen. Die Erfindung betrifft auch einen Katheter mit einer solchen flexible Leiterplatte.

### Stand der Technik

Aus dem Stand der Technik sind unterschiedlichste Bauformen von Kathetern bekannt, welche ihrer medizinischen Anwendung entsprechend aufgebaut sind. Darunter sind auch elektrodentragende Katheter wie sie beispielsweise in der DE 694 01 562 T2 beschrieben sind. Auch bekannt sind sogenannte Mappingkatheter, beispielsweise zur 3-dimensionalen Erfassung der Anatomie einer Herzkammer, welche eine hohe Anzahl an Elektroden aufweisen müssen.

Bei den aus dem Stand der Technik bekannten Kathetern entstehen hohe Produktionskosten, weil die zu den Elektroden führenden Leiterbahnen aufwändig im Katheterrohr untergebracht werden müssen. Weiter problematisch ist, dass sich auf Grund der verwendeten Materialien eine Beschränkung bzgl. des Biegeradius des Katheters ergeben kann und damit eine Einschränkung in dessen Einsatz. Der Einsatz flexibler Leiterbahnen ist daher vorteilhaft. Flexible Leiterbahnen werden auch als Flex Circuits, Flexible Printed Circuits oder Flexible Printed Circuit Boards (flexible PCB) bezeichnet.

Gemäß der US 2006/0244177 A1 wird eine lange Leiterbahn für einen Katheter geschaffen durch Heraustrennen einer mäanderförmigen Bahn aus einer Grundplatte und nachfolgendes Falten entlang von je zwei Faltkanten im jeweiligen Umlenkungsbereich. Nachteilig bei dieser Lösung ist, dass die Leiterbahn im Bereich der Faltungen eine größere Breite aufweist. Diese größere Breite ist für die Verwendung im beengten Innern eines Hohlkatheters problematisch. Weiter nachteilig ist, dass im Bereich der Faltungen drei Lagen der Leiterbahn aufeinander liegen. Diese größere Dicke ist für die Verwendung im beengten Innern eines Hohlkatheters ebenfalls problematisch.

### Aufgabenstellung

Aufgabe der vorliegenden Erfindung ist es eine flexible Leiterplatte zu schaffen, welche eine große Länge und gleichzeitig besonders kompakte Abmaße in ihrer Breite und Dicke aufweist und so die Nachteile des Standes der Technik zumindest teilweise behebt.

### Technische Lösung

Gelöst wird diese Aufgabe durch eine flexible Leiterplatte in der Form eines Streifens, wie sie nachfolgend beschrieben und beansprucht ist.

Erfindungsgemäß wurde als vorteilhaft erkannt derart Faltungen in Umlenkbereichen vorzusehen, dass nach erfolgter Faltung maximal zwei Lagen des Streifens aufeinander aufliegen.

Die erfindungsgemäße flexible Leiterplatte aus einem einteiligen Streifen dient zur Verwendung in einem Katheter. Der Streifen weist mehrere sich winkelig aneinander anschließende Abschnitte auf, derart, dass Umlenkungen des Streifens erfolgen, und der Streifen weist Faltkanten in dem Streifen im Bereich der Umlenkungen auf. Durch Falten entlang der Faltkanten wird ein linearer gerader, bandförmiger Streifen gebildet. Vor dem Falten liegen die winkeligen Abschnitte alle in einer Ebene.
In vorteilhafter Weise liegen im Bereich von drei Umlenkungen zwei Faltkanten vor und es liegen nach erfolgtem Falten maximal zwei Lagen des Streifens aufeinander auf. In anderen Worten: der Bereich einer jeweiligen Umlenkung wird durch zwei winklige Abschnitte gebildet. Ein jeweiliger winkliger Abschnitt ist - vor der Faltung - winklig relativ zu den Seitenkanten des ungefalteten Bereichs des Streifens. Durch die Faltung werden die winkligen Abschnitte teilweise aufeinander gelegt, sodass ein gerader bandförmiger Streifen gebildet wird. Es liegen jedoch maximal zwei Lagen des Streifens aufeinander, sodass der Streifen auch nach erfolgtem Falten eine geringe Dicke aufweist und gut in beengten Platzverhältnissen verwendet werden kann.
Durch mehrfaches Anwenden der beschriebenen Umlenkung und Faltung über die Länge des Streifens kann ein besonders langer Streifen geschaffen werden.

Bei der flexible Leiterplatte schließen *die Seitenkante des Streifens unmittelbar vor der ersten Umlenkung* und *die Seitenkante des Streifens unmittelbar nach der dritten Umlenkung* einen Umlenkungswinkel β ein. Beim Umlenkungswinkel β handelt es sich also um den Winkel der Gesamtumlenkung von drei sich aneinander anschließenden Umlenkungen.

In Weiterbildung der flexiblen Leiterplatte sind die Seitenkanten des Streifens überwiegend parallel zueinander, und insbesondere weist der Streifen im Bereich zwischen der ersten Umlenkung und der dritten Umlenkung, also im Bereich der winkligen Abschnitte, vor erfolgtem Falten ebenfalls parallele Seitenkanten auf.

In besonders vorteilhafter und daher bevorzugter Weiterbildung der flexiblen Leiterplatte weist der Streifen mit seinen winkligen Abschnitten und Umlenkungen vor erfolgten Faltungen in der Draufsicht ein mäanderförmiges Muster auf. Dies ermöglicht, dass aus einer das Grundmaterial bildenden Platte ein besonders langer einteiliger Streifen gefertigt werden kann. Bevorzugt liegt der Umlenkwinkel β im Bereich 0 < β ≤ 120°. Denkbar ist auch eine Sonderform mit einem Umlenkwinkel β von 180°. Wird als Umlenkwinkel β =90° gewählt, so genügen zwei hintereinander angeordnete Umlenkbereiche, um "um die Kurve" zu kommen und den Streifen nach einer Gesamtumlenkung von 180° fortzusetzen. Wird als Umlenkwinkel β =180° gewählt, so genügt schon ein Umlenkbereich, um "um die Kurve" zu kommen. In beiden Fällen wird eine gute Ausnutzung des Grundmaterials bei geringer Anzahl an erforderlichen Faltungen ermöglicht.

In einer vorteilhaften Ausgestaltung der flexiblen Leiterplatte gilt für einen Winkel a:
- der Winkel a zwischen *der Mittellinie des Streifens vor dem Bereich der ersten Umlenkung* und zwischen *der Außenkante des Streifens nach der ersten Umlenkung* entspricht
- dem Winkel α zwischen *der Mittellinie des Streifens vor dem Bereich der ersten Umlenkung* und zwischen *der ersten Faltkante* und entspricht auch
- dem Winkel α *zwischen der Außenkante im Bereich zwischen zweiter und dritter Umlenkung* und zwischen *der zweiten Faltkante.*

In Weiterbildung der flexiblen Leiterplatte entspricht der Umlenkungswinkel β, also der Winkel β zwischen der Außenkante des Streifens unmittelbar vor der ersten Umlenkung und zwischen der Außenkante des Streifens unmittelbar nach der dritten Umlenkung, dem Winkel β zwischen *der Außenkante des Streifens unmittelbar vor zweiten Umlenkung* und *der Verlängerung der Außenkante des Streifens unmittelbar nach der zweiten Umlenkung.*

In besonders vorteilhafter Weiterbildung der vorbeschriebenen flexiblen Leiterplatte gilt der folgende Zusammenhang: der Winkel α ist halb so groß wie der Winkel β, also α = β / 2.

Weiter gilt, dass der Streifen vor und nach den drei Umlenkungen jeweils eine Breite b aufweist und der Streifen zwischen erster und dritter Umlenkung mindestens eine Breite von d = b · cos α aufweist.
Durch eine entsprechende Wahl des Winkels α bzw. β kann also die Breite d beeinflusst werden. Ist eine bestimmte Breite d erforderlich, beispielsweise um eine gewisse Anzahl von Leiterbahnen auf dem Streifen unterzubringen, kann β=45° gewählt werden. Bei größeren Winkeln β wird die Breite d geringer ausfallen.

Die Erfindung betrifft auch einen Katheter mit einer flexiblen Leiterplatte wie vorstehend beschrieben.

Der Katheter ist bevorzugt in Form eines Hohlkatheter ausgebildet. Der Katheter hat die Form eines länglichen, hohlen und flexiblen Rohres, welches auch als Schlauch bezeichnet werden könnte. Der Katheter besitzt mindestens eine Elektrode zur Durchführung einer Diagnose, beispielsweise einer Messung wie einer elektrophysiologischen Untersuchung, oder zur Durchführung einer Therapie, beispielsweise einer Operation wie einer Katheterablation, jeweils im menschlichen Körper. Die mindestens eine Elektrode ist an einem Endbereich des Rohres angeordnet und am anderen Endbereich des Rohres ist ein elektrischer Anschluss angebracht zum elektrischen Verbinden des Katheters mit einer Steuereinrichtung, sodass eine stromleitende und datenübertragungstechnische Verbindung zwischen der mindestens einen Elektrode und dem elektrischen Anschluss besteht.
In vorteilhafter Weise sind die mindestens eine Elektrode und der elektrische Anschluss mittels Leiterbahnen auf mindestens einer flexiblen Leiterplatte, welche ausgeführt ist wie obenstehend beschrieben, elektrisch leitend miteinander verbunden. Die flexible Leiterplatte ist dazu in oder auf dem Rohr angeordnet.

Die Erfindung betrifft auch die Verwendung eines wie obenstehend beschriebenen Katheters als Herzkatheter, welcher in der Diagnose oder Therapie des menschlichen Herzens zum Einsatz gelangt.

Die beschriebene Erfindung und die beschriebenen vorteilhaften Weiterbildungen der Erfindung stellen auch in Kombination miteinander - soweit dies technisch sinnvoll ist - vorteilhafte Weiterbildungen der Erfindung dar.

Hinsichtlich weiterer Vorteile und in konstruktiver und funktioneller Hinsicht vorteilhafter Ausgestaltungen der Erfindung wird auf die Unteransprüche sowie die Beschreibung von Ausführungsbeispielen unter Bezugnahme auf die beiliegenden Figuren verwiesen.

### Ausführungsbeispiel

Die Erfindung soll anhand beigefügter Figuren noch näher erläutert werden. Einander entsprechende Elemente und Bauteile sind in den Figuren mit gleichen Bezugszeichen versehen. Zugunsten einer besseren Übersichtlichkeit der Figuren wurde auf eine maßstabsgetreue Darstellung verzichtet.
Es zeigen in schematischer Darstellung
- Fig. 1a: eine erste Variante einer flexiblen Leiterplatte in ungefaltetem Zustand und in gefaltetem Zustand
- Fig. 1b: eine zweite Variante einer flexiblen Leiterplatte in ungefaltetem Zustand
- Fig. 1c: eine dritte Variante einer flexiblen Leiterplatte in ungefaltetem Zustand
- Fig. 2a: die erste Variante einer flexiblen Leiterplatte mit Maßangaben
- Fig. 2b: die zweite Variante einer flexiblen Leiterplatte mit Maßangaben
- Fig. 2c: die dritte Variante einer flexiblen Leiterplatte mit Maßangaben
- Fig. 2d: eine vierte Variante einer flexiblen Leiterplatte mit Maßangaben
- Fig. 3: eine flexible Trägerplatte, aus welcher die flexiblen Leiterplatten herausgetrennt werden

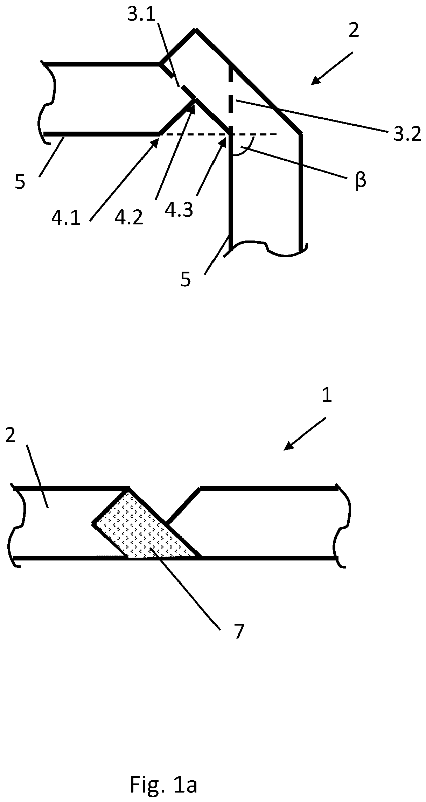

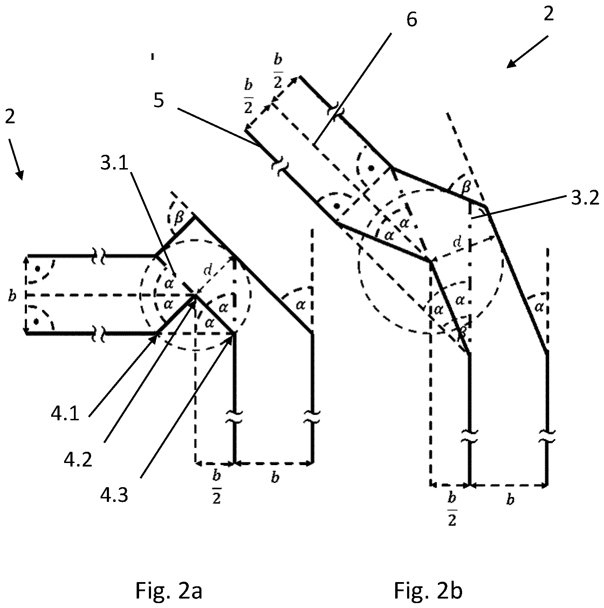

Fig. 1a zeigt eine erste Variante einer flexiblen Leiterplatte 1, in der oberen Abbildung in ungefaltetem Zustand und in der unteren Abbildung in gefaltetem Zustand.
Die flexible Leiterplatte 1 besitzt einem Streifen 2, welcher in den Abbildungen ausschnittsweise dargestellt ist, und der für einen Katheter verwendet werden kann. Leiterbahnen auf der Leiterplatte 1 sind zur besseren Übersichtlichkeit nicht dargestellt.
Der Streifen 2 weist mehrere sich winkelig aneinander anschließende Abschnitte auf, derart, dass drei Umlenkungen 4.1, 4.2, 4.3 des Streifens 2 erfolgen. Die Seitenkante 5 des Streifens 2 vor der ersten Umlenkung 4.1 und die Seitenkante 5 des Streifens 2 nach der dritten Umlenkung 4.3 schließen einen Umlenkungswinkel β ein, mit β = 90°. Formgebung und Geometrie des Streifens werden anhand der Figur 2a noch näher erläutert.
Es sind zwei Faltkanten 3.1, 3.2 in dem Streifen 2 im Bereich der Umlenkungen 4.1, 4.2, 4.3 vorgesehen, wobei durch Falten entlang der Faltkanten 3.1, 3.2 (gestrichelt dargestellt) ein linearer gerader Streifen 2 gebildet ist, wie er in der unteren Abbildung dargestellt ist.
Die Ausgestaltung von Umlenkungen 4.1, 4.2, 4.3, Form des Streifens 2 und Lage der Faltkanten 3.1, 3.2 ist derart gewählt, dass nach erfolgtem Falten maximal zwei Lagen des Streifens 2 aufeinander aufliegen. Dieser Bereich 7 mit zwei aufeinander aufliegenden Lagen ist in der unteren Abbildung punktiert.

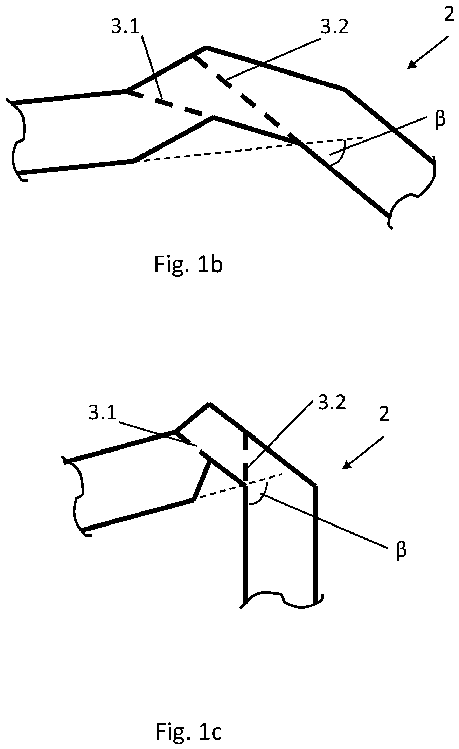

Fig. 1b zeigt eine zweite Variante einer flexiblen Leiterplatte in ungefaltetem Zustand mit einem Umlenkungswinkel β von 45°, wobei durch Falten entlang der Faltkanten 3.1, 3.2 ein linearer gerader Streifen 2 gebildet ist (nicht dargestellt).

Bei Varianten einer flexiblen Leiterplatte mit einem Umlenkungswinkel 0 < β < 90° sind Form und Aufbau ähnlich.

Fig. 1c zeigt eine dritte Variante einer flexiblen Leiterplatte in ungefaltetem Zustand mit einem Umlenkungswinkel β von 105°, wobei durch Falten entlang der Faltkanten 3.1, 3.2 ein linearer gerader Streifen 2 gebildet ist (nicht dargestellt).

Bei Varianten einer flexiblen Leiterplatte mit einem Umlenkungswinkel 90 < β ≤ 120° sind Form und Aufbau ähnlich.

Fig. 2a zeigt die erste Variante einer flexiblen Leiterplatte aus Fig. 1a mit Maßangaben.

Fig. 2b zeigt die zweite Variante einer flexiblen Leiterplatte aus Fig. 2a mit Maßangaben.

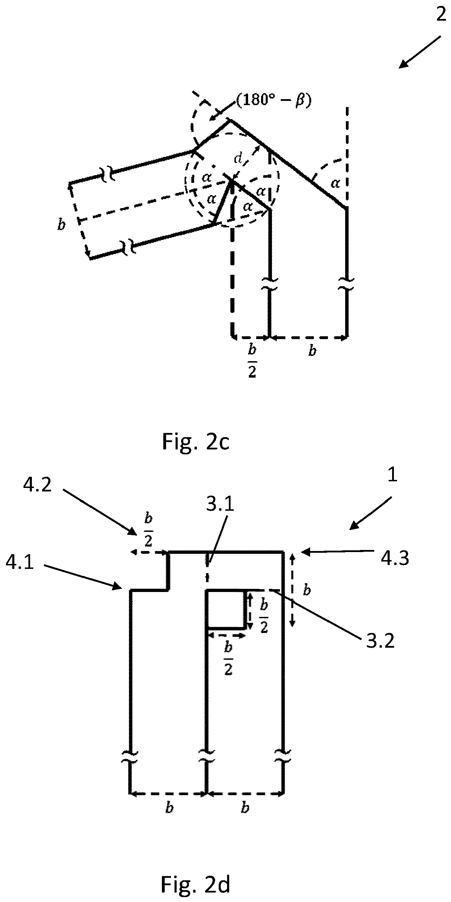

Fig. 2c zeigt die dritte Variante einer flexiblen Leiterplatte aus Fig. 1c mit Maßangaben.

Für alle Varianten gemäß Fig. 2a-2c gilt:
- der Winkel α zwischen der Mittellinie 6 des Streifens 2 vor dem Bereich der ersten Umlenkung 4.1 und zwischen der Außenkante 5 des Streifens 2 nach der ersten Umlenkung 4.1 entspricht in seiner Größe
- dem Winkel α zwischen der Mittellinie 6 des Streifens 2 vor dem Bereich der ersten Umlenkung 4.1 und zwischen der ersten Faltkante 3.1 und entspricht in seiner Größe
- dem Winkel α zwischen der Außenkante 5 im Bereich zwischen zweiter und dritter Umlenkung 4.2, 4.3 und zwischen der zweiten Faltkante 3.2.
Alle gleichgroßen Winkel sind mit dem Bezugszeichen α gekennzeichnet.

Der Umlenkungswinkel β entspricht dem Winkel β zwischen der Außenkante 5 des Streifens 2 unmittelbar vor der zweiten Umlenkung 4.2 und der gestrichelt gezeichneten Verlängerung der Außenkante 5 des Streifens 2 unmittelbar nach der zweiten Umlenkung 4.2.

Weiter gilt in den dargestellten Ausführungsbeispielen: α = β / 2.

Der Streifen 2 vor und nach den drei Umlenkungen 4.1, 4.4, 4.3 weist jeweils eine Breite b auf, welche auch als nominale Breite bezeichnet werden kann. Zwischen erster und dritter Umlenkung 4.1, 4.3 weist der Streifen 2 eine reduzierte Breite d auf, welche für alle denkbaren Winkel α (mit 0 < α <90°) kleiner als die nominale Breite b ist, nämlich eine Breite von d = b · cos α.

Fig. 2d zeigt eine vierte Variante einer flexiblen Leiterplatte mit Maßangaben mit einem Umlenkungswinkel β von 180°. Diese stellt einen Sonderfall dar.

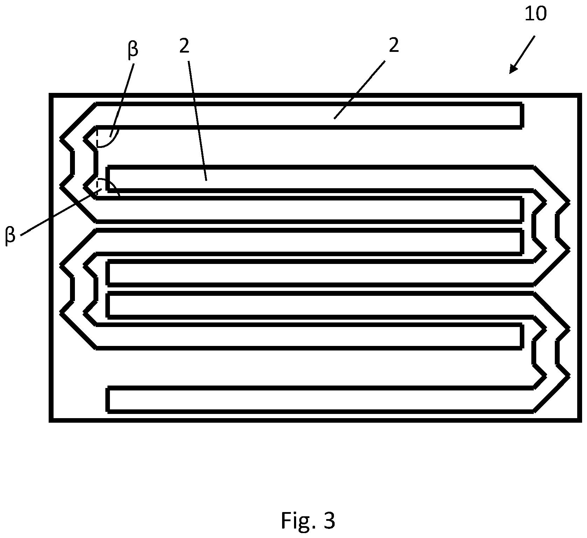

Fig. 3 zeigt eine flexible Trägerplatte 10 als Grundmaterial, aus welcher die flexiblen Leiterplatten 1 herausgetrennt werden. Der Streifen 2 weist mit seinen winkligen Abschnitten und Umlenkungen 4.1, 4.2, 4.3 vor einem Heraustrennen in der Draufsicht ein mäanderförmiges Muster auf. Dies ermöglicht, dass aus einer das Grundmaterial bildenden Platte 10 ein besonders langer einteiliger Streifen 2 gefertigt werden kann. Wird - wie im dargestellten Beispiel - als Umlenkwinkel β =90° gewählt, so genügen zwei hintereinander angeordnete Umlenkbereiche, um "um die Kurve" zu kommen.

Ein Verfahren zur Fertigung einer flexiblen Leiterplatte 1 in Form eines langen Streifens 2, insbesondere für einen Katheter kann mit folgenden Schritten ablaufen:
a. Bereitstellen einer flexiblen Trägerplatte 10.
b. Aufbringen von Leiterbahnen (nicht dargestellt) auf die flexiblen Trägerplatte 10.
c. Heraustrennen eines Streifens 2 aus der Trägerplatte 10
d. Falten des Streifens 2 an den vorgesehenen Faltkanten 3.1, 3.2 zur Herstellung einer flexiblen Leiterplatte 1.

Das Heraustrennen kann durch Stanzen oder Schneiden, insbesondere Brennschneiden oder Strahlschneiden erfolgen, wie beispielsweise Wasserstrahlschneiden oder Laserstrahlschneiden.

### Bezugszeichenliste

- 1: Flexible Leiterplatte
- 2: Streifen
- 3.1: 1. Faltkante
- 3.2: 2. Faltkante
- 4.1: 1. Umlenkung
- 4.2: 2. Umlenkung
- 4.3: 3. Umlenkung
- 5: Seitenkante des Streifens
- 6: Mittellinie des Streifens
- 7: Bereich des Streifens mit zwei Lagen

- 10: Platte (Grundmaterial)

- b: Nominale Breite des Streifens
- d: Minimale Breite des Streifens

- α: Winkel
- β: Umlenkungswinkel

## Patentansprüche

1. Flexible Leiterplatte (1) aus einem Streifen (2) für einen Katheter, wobei der Streifen (2) mehrere sich winkelig aneinander anschließende Abschnitte aufweist, derart, dass Umlenkungen (4.1, 4.2, 4.3) des Streifens (2) erfolgen, mit Faltkanten (3.1, 3.2) in dem Streifen (2) im Bereich der Umlenkungen (4.1, 4.2, 4.3), wobei durch Falten entlang der Faltkanten (3.1, 3.2) ein linearer gerader Streifen gebildet ist, **dadurch**
**gekennzeichnet, dass**
im Bereich von drei Umlenkungen (4.1, 4.2, 4.3) zwei Faltkanten (3.1, 3.2) vorliegen und maximal zwei Lagen des Streifens (2) aufeinander aufliegen.

2. Flexible Leiterplatte nach Anspruch 1, **dadurch gekennzeichnet, dass**
die Seitenkante (5) des Streifens (2) vor der ersten Umlenkung (4.1) und die Seitenkante (5) des Streifens (2) nach der dritten Umlenkung (4.3) einen Umlenkungswinkel β einschließen.

3. Flexible Leiterplatte nach einem der vorangehenden Ansprüche **dadurch**
**gekennzeichnet, dass**
die Seitenkanten (5) des Streifens (2) parallel zueinander sind, und dass insbesondere der Streifen (2) im Bereich zwischen der ersten Umlenkung (4.1) und der dritten Umlenkung (4.3) parallele Seitenkanten (2) aufweist.

4. Flexible Leiterplatte nach einem der vorangehenden Ansprüche, **dadurch**
**gekennzeichnet, dass**
der Streifen (2) mit seinen winkligen Abschnitten und Umlenkungen (4.1, 4.2, 4.3) ein mäanderförmiges Muster aufweist.

5. Flexible Leiterplatte nach einem der vorangehenden Ansprüche, **dadurch**
**gekennzeichnet, dass**
- der Winkel α zwischen der Mittellinie (6) des Streifens (2) vor dem Bereich der ersten Umlenkung (4.1) und zwischen der Außenkante (5) des Streifens (2) nach der ersten Umlenkung (4.1)
- dem Winkel α zwischen der Mittellinie (6) des Streifens (2) vor dem Bereich der ersten Umlenkung (4.1) und zwischen der ersten Faltkante (3.1) entspricht und
- dem Winkel α zwischen der Außenkante (5) im Bereich zwischen zweiter und dritter Umlenkung (4.2, 4.3) und zwischen der zweiten Faltkante (3.2) entspricht.

6. Flexible Leiterplatte nach einem der Ansprüche 2 - 5, **dadurch**
**gekennzeichnet, dass**
der Umlenkungswinkel β dem Winkel β zwischen der Außenkante (5) des Streifens (2) unmittelbar vor der zweiten Umlenkung (4.2) und der Verlängerung der Außenkante (5) des Streifens (2) unmittelbar nach der zweiten Umlenkung (4.2) entspricht.

7. Flexible Leiterplatte nach Anspruch 5 und 6 **dadurch gekennzeichnet, dass** gilt α = β / 2.

8. Flexible Leiterplatte nach Anspruch 5 und 6 oder nach Anspruch 7
**dadurch gekennzeichnet, dass**
der Streifen (2) vor und nach den drei Umlenkungen (4.1, 4.4, 4.3) jeweils eine Breite b aufweist und der Streifen (2) zwischen erster und dritter Umlenkung (4.1, 4.3) mindestens eine Breite von d = b · cos α aufweist.

9. Katheter, insbesondere Hohlkathether, mit einer flexiblen Leiterplatte (1) nach einem der vorangehenden Ansprüche.

10. Katheter nach Anspruch 9, **dadurch gekennzeichnet, dass** der Katheter in Form eines länglichen, hohlen und flexiblen Rohres mit mindestens einer Elektrode ausgebildet ist zur Durchführung einer Diagnose oder Therapie im menschlichen Körper, wobei die mindestens eine Elektrode an einem Endbereich des Rohres angeordnet ist und am anderen Endbereich des Rohres ein elektrischer Anschluss angebracht ist, zum elektrischen Verbinden des Katheters mit einer Steuereinrichtung, und dass die mindestens eine Elektrode und der elektrische Anschluss mittels Leiterbahnen auf der flexiblen Leiterplatte (1) elektrische leitend miteinander verbunden sind, wobei die flexible Leiterplatte (1) in oder auf dem Rohr angeordnet ist.
